# FASCICULE DE BREVET EUROPEEN

(11) **EP 0 938 311 B1**
(45) Date de publication et mention de la délivrance du brevet: **05.02.2003**
(21) Numéro de dépôt: 97938939.2
(22) Date de dépôt: 12.08.1997
(51) Int. Cl.: A61K 31/415, A61P 25/14

(54) **UTILISATION DE L'EFAROXAN POUR LA FABRICATION DE MEDICAMENT DESTINE AU TRAITEMENT DE LA MALADIE DE HUNTINGTON**
VERWENDUNG VON EFAROXAN ZUR HERSTELLUNG EINES ARZNEIMITTELS ZUR BEHANDLUNG VON HUNTINGTON KRANKHEIT
USE OF EFAROXAN FOR PRODUCING MEDICINE FOR TREATING HUNTINGTON DISEASE

(30) Priorité: 12.08.1996 FR 9610118
(43) Date de publication de la demande: 01.09.1999
(73) Titulaire: PIERRE FABRE MEDICAMENT, 92100 Boulogne-Billancourt (FR)
(72) Inventeur: MARIEN, Marc, F-81100 Castres (FR); MARTEL, Jean-Claude, F-81100 Castres (FR); COLPAERT, Francis, F-81100 Castres (FR); IMBERT, Thierry, F-81290 Viviers-les-Montagnes (FR)
(74) Mandataire: Ahner, Francis
(86) Numéro de dépôt international: FR9701480
(87) Numéro de publication internationale: WO98006393

(56) Documents cités:
- WO-A-95/01791
- G. OLMOS ET AL.: "Imidazoli(di)ne compounds interact with the phencyclidine site of NMDA receptors in the rat brain." EUR. J. PHARMACOL., vol. 310, no. 2/3, 1996, pages 273-276, XP000670832
- H.C. JACKSON ET AL.: "Exploring the pharmacology of the pro-convulsant effects of alpha-2-adrenoceptor antagonists in mice." PSYCHOPHARMACOLOGY, vol. 105, no. 4, 1991, pages 558-562, XP000670893

## Description

La présente invention concerne l'utilisation de l'Efaroxan composé de formule suivante : 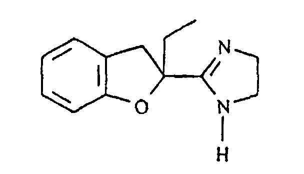 ainsi que de ses sels thérapeutiquement acceptables, de sa forme racémique ou de ses isomères optiquement actifs, pour la préparation d'un médicament destiné au traitement de la maladie de Huntington.

La présente invention a pour objet l'utilisation de l'Efaroxan pour obtenir un médicament neuroprotecteur, destiné au traitement de la maladie d'Huntington et de sa progression.

La maladie d'Huntington est considérée comme une conséquence pathologique de lésions des systèmes Gabaergiques et cholinergiques au niveau du striatum provoqués par des substances excitotoxiques sur le complexe : canaux ioniques - récepteur NMDA.

Les manifestations cliniques de la maladie d'Huntington sont des troubles moteurs notamment des mouvements anormaux choreiformes qui s'aggravent progressivement et accompagnés par la suite de bradykinésie et de rigidité musculaire, ainsi que par des problèmes neuropsychiatriques comme la dépression, la tendance suicidaire, les troubles de la personnalité et cognitifs. Ces troubles très tôt se manifestent par une perte de l'habileté visuospatiale qui peut précéder de quelques années les mouvements choreiformes, avec une perte cognitive telle qu'on l'observe chez les patients ayant des atteintes du lobe frontal.

Un aspect particulier de la maladie est la perte de mémoire, en particulier la fonction de rappel. D'un point de vue neuropathologique une caractéristique de la maladie est une nette atrophie du corps strié où il y a atteinte des systèmes efférents, des interneurones, ainsi qu'une gliose marquée. A un niveau moindre on observe le même phénomène dans le globus pallidus, le thalamus, la substance noire, le locus coeruleus et le cortex.

Actuellement aucun traitement n'est disponible pour soigner ou retarder l'évolution de la maladie d'Huntington.

Il est connu que l'Efaroxan : 2-[2-(2-ethyl-2,3-dihydrobenzofuranyl)]-2-imidazoline possède des propriétés antagonistes sur les récepteurs α₂ adrénergiques. Ce composé est décrit dans la demande de brevet GB2102422 ainsi que son application thérapeutique en tant que médicament antidépresseur et antimigraineux. Ce composé est également décrit dans la demande de brevet WO92/05171 où est mise en évidence l'action de l'énantiomère lévogyre pour traiter le diabète, comme agent bloqueur des canaux potassiques.

Nos brevets WO94/00715 et WO94/00841 relatent également l'utilisation de l'Efaroxan dans le traitement de la maladie de Parkinson ainsi que dans la maladie d'Alzheimer.

La présente invention concerne l'utilisation de l'Efaroxan pour la préparation d'un médicament destiné au traitement de la maladie d'Huntington.

Par Efaroxan, on entend le composé de formule : 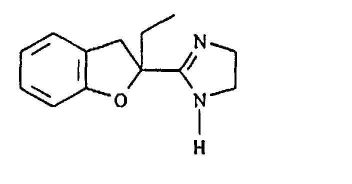 ses sels thérapeutiquement acceptables, son racémique ou ses isomères optiquement actifs.

### Etude pharmacologique :

L'acide quinolinique, métabolique endogène du tryptophane agit comme un puissant agoniste sur le récepteur NMDA (Eur. J. Pharm (1981), 72, 411). Son injection dans le striatum chez le rat et le singe provoque des modifications neurochimiques et morphologiques similaires à celles observées dans la maladie d'Huntington (Life Sci. (1984), 35, 19) et se traduisent par un déficit de l'habileté spatiale, des capacités cognitives et du rappel. Cette atteinte du striatum explique ainsi la maladie d'Huntington (Behav. Brain Res. (1987), 24, 125).

Dans les cerveaux atteints par la maladie, le taux d'enzyme de synthèse de l'acide quinolinique, 3-hydroxyanthranilate oxygenase (3-HAO), est élevé. De plus, le niveau de l'acide kynurenique (un autre métabolite du tryptophane et un antagoniste de l'acide quinolinique ) est abaissé, impliquant ainsi une mauvaise balance entre ces deux acides quinolinique et kynurenique dans les cerveaux atteints de la maladie de Huntington. (Pharmacol. Rev. 1993, 45, 309).

Ainsi une intervention pharmacologique capable de restaurer les fonctions du striatum dans ce modèle, peut avoir une utilité dans le traitement des manifestations motrices et cognitives de la maladie d'Huntington chez l'homme (J. Neuroscience (1988), 8, 3901 et Pharmacol. Rev. (1993), 45, 309).

Les paramètres mesurés sont :
1°) L'activité de la choline acetyltranférase (ChAT) dans le striatum, marqueur des neurones cholinergique dans cette partie du cerveau. Une réduction de l'activité de la ChAT, suivant une injection intrastriatale d'acide quinolinique est une mesure quantitative de la perte de neurones cholinergiques striataux, et est correllée avec l'importance de la lésion neurotoxique dans cette partie du cerveau. Dans ce modèle, la capacité pour une drogue d'atténuer la perte d'activité de la ChAT, induite par l'acide quinolinique, est considérée comme une indication d'un effet protecteur ou restaurateur de l'intégrité du système cholinergique striatal. Ce modèle utilisé est décrit par T.W. Stone (Pharmacol. Rev. 1993. 45, 309), M.F. Beal et al. (J. Neurosci.1988, 8, 3901), et M. Miyamoto et J.T. Coyle (Exptl. Neurol. 1990, 108, 38.).
2°) Le comportement de rotation induit par l'apomorphine, indicateur du dysfonctionnement du système striatal efférent, modèle décrit par C.J. Pycock, (Neuroscience, 1980, 5, 461). Cette publication passe en revue les évidences qui démontrent que des lésions unilatérales dans le striatum par des exitotoxines, comme l'acide quinolinique, provoquent un comportement de rotations (ipsilatérales) chez les animaux ayant reçu de l'apomorphine, et que l'intensité de ces rotations est en relation avec l'étendue de la lésion. La capacité pour une drogue de réduire le nombre de rotations induites par l'apomorphine est considérée comme indicative d'un effet protecteur ou restaurateur de l'intégrité du fonctionnement des neurones striataux efférents.

Les résultats sont les suivants :

### 1°) Activité ChAT :

La ChAT est diminuée à 26 ± 8% (mean ± SEM) de l'activité normale (mesure dans le striatum non lésé), 2 semaines après l'injection unilatérale intrastriatale, chez le rat, d'acide quinolinique (150 nmoles).

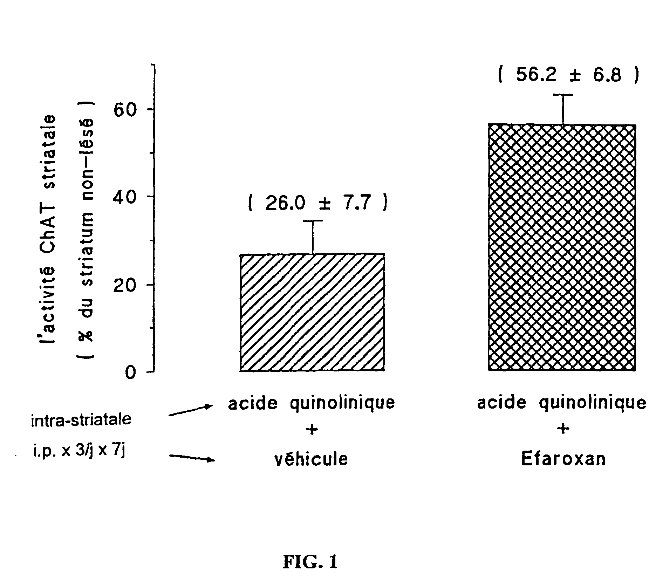

L'administration 3 fois par jour pendant 7 jours de 0.63 mg/kg d'Efaroxan, à partir du même jour de l'injection de l'acide quinolinique, atténue partiellement cette perte d'activité de la ChAT. Dans ce cas, l'activité est diminuée seulement à 56 ± 7% (mean ± SEM) de l'activité normale, comme on peut le voir dans la figure 1 annexée qui illustre la perte de l'activité de la ChAT induite par l'acide quinolinique dans le striatum du rat et traduit donc de l'effet protecteur exercé.

### 2°) Test comportemental de rotation :

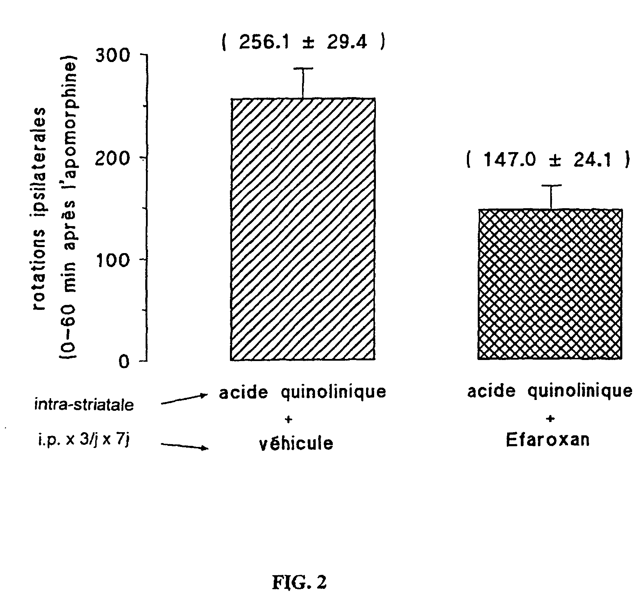

Chez les animaux lésés avec l'acide quinolinique comme précédemment, une injection de 0.63 mg/kg d'apomorphine, deux semaines plus tard induit une rotation ipsilatérale (256 ± 29 rotations/h, mean ± SEM). L'administration de 0,63 mg/kg d'éfaroxan comme précédemment, 3 fois par jour pendant 7 jours diminue de 43% le nombre de ces rotations ipsilatérales (à 147 ± 24 rotations/h, mean ± SEM), comme le montre la figure 2 annexée illustrant les rotations induites par l'apomorphine chez les rats lésés par l'acide quinolique et démontre l'atténuation de l'effet par l'Efaroxan.

Les résultats précédents montrent l'intérêt de l'Efaroxan pour diminuer les effets deletères exitotoxiques de l'acide quinolinique dans le striatum, et son intérêt dans la prévention des troubles neurotoxiques où les récepteurs au glutamate sont impliqués.

L'Efaroxan présente ainsi un intérêt pour être utilisé en tant que médicament pour traiter, ou retarder ou d'empêcher l'évolution de la maladie d'Huntington.

### Etude Galénique :

Les compositions pharmaceutiques faisant partie de l'invention, sont administrées par voie orale chez l'homme, en une ou plusieur fois, sous forme de gélules ou de comprimés dosés de 1 à 200 mg de principe actif, plus particulièrement de 7, 20, 30 et 40 mg par comprimé, ou par voie intraveineuse sous forme de soluté injectable dosée de 0.1 à 10 mg d'Efaroxan.

### Etude clinique :

L'Efaroxan a été administré à la dose de 30 mg 3 fois par jour à un groupe de malades présentant la symptomatologie de la maladie d'Huntington. Les résultats montrent une amélioration comportementale dans environ 30 % des cas.

## Revendications

1. Utilisation de l'Efaroxan, composé de formule: 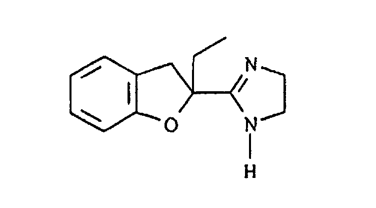 de ses sels thérapeutiquement acceptables, son racémique, ou ses isomères optiquement actifs, pour la préparation d'un médicament destiné au traitement de la maladie d'Huntington.

2. Utilisation de l'Efaroxan selon la revendication 1 en ce qu'il se présente sous forme d'une composition pharmaceutique administrable à l'homme, contenant de 7 à 40 mg de principe actif.

## Claims

1. Use of Efaroxan, the compound of formula: 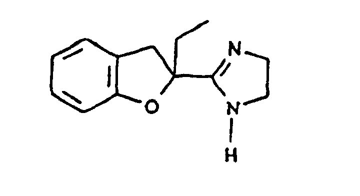 its therapeutically acceptable salts, its racemic mixture or its optically active isomers, for the preparation of a medicinal product for the treatment of Huntington's disease.

2. Use of Efaroxan according to Claim 1, **characterized in that** it is in the form of a pharmaceutical composition which can be administered to man, containing from 7 to 40 mg of active principle.

## Patentansprüche

1. Verwendung von Efaroxan, Verbindung der Formel : 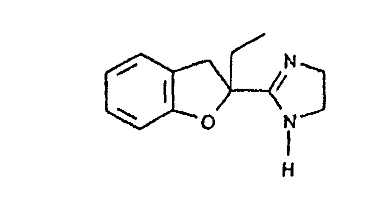 von dessen therapeutisch annehmbaren Salzen, dessen Racemat oder dessen optisch aktiven Isomeren für die Herstellung eines Arzneimittels, das für die Behandlung von Huntington Krankheit bestimmt ist.

2. Verwendung von Efaroxan nach Anspruch 1, das in Form einer einer an den Menschen verabreichbaren pharmazeutischen Zusammensetzung, die 7 bis 40 mg Wirkstoff enthält, vorliegt.
